# EUROPÄISCHE PATENTSCHRIFT

(11) **EP 4 009 917 B1**
(45) Veröffentlichungstag und Bekanntmachung des Hinweises auf die Patenterteilung: **30.04.2025**
(21) Anmeldenummer: 20750259.2
(22) Anmeldetag: 31.07.2020
(51) Int. Cl.: A61F 2/66, A61F 2/68, A61F 2/50, A61F 2/74

(54) **VERFAHREN ZUM STEUERN EINES PROTHESENFUSSES**
METHOD FOR CONTROLLING A PROSTHETIC FOOT
MÉTHODE DE CONTRÔLE D'UNE PROTHÈSE DE PIED

(30) Priorität: 09.08.2019 DE 102019121595
(43) Veröffentlichungstag der Anmeldung: 15.06.2022
(73) Patentinhaber: Ottobock SE & Co. KGaA, 37115 Duderstadt (DE)
(72) Erfinder: BELTRAN ULLAURI, Jessica, Gabriela, 37412 Herzberg (DE); BOHLAND, Andreas, 1070 Wien (AT); SEYR, Martin, 1070 Wien (AT); PAPPE, Alexander, 1180 Wien (AT)
(74) Vertreter: Gramm, Lins & Partner Patent- und Rechtsanwälte PartGmbB
(86) Internationale Anmeldenummer: PCT/EP2020/071712
(87) Internationale Veröffentlichungsnummer: WO 2021/028244

(56) Entgegenhaltungen:
- EP-B1- 2 087 858
- DE-A1- 102014 010 938
- DE-T2- 60 309 685
- DE-T2- 60 309 685
- DE-U1- 202016 107 294
- DE-U1- 202016 107 294
- US-A1- 2002 138 153
- US-A1- 2016 278 947

## Beschreibung

Die Erfindung betrifft ein Verfahren zum Steuern eines Prothesenfußes, der ein Fußteil und ein Unterschenkelteil aufweist, die mittels eines Gelenkes miteinander verbunden sind, das eine Plantarflexion und eine Dorsalflexion erlaubt, wobei das Gelenk eine einstellbare Dämpfung aufweist.

Derartige Prothesenfüße sind aus dem Stand der Technik seit langem bekannt. Das Gelenk, das den Fußteil mit dem Unterschenkelteil verbindet, bildet das Knöchelgelenk des Prothesenfußes. Es handelt sich in der Regel um ein Schwenkgelenk, das eine Verschwenkung des Fußteils relativ zum Unterschenkelteil um eine einzige Schwenkachse erlaubt. Es sind jedoch auch mehrachsige Schwenkgelenke oder andere Anordnungen möglich. Bei Prothesenfüßen der hier beschriebenen Art erlaubt das Gelenk eine Plantarflexion und eine Dorsalflexion. Die Dorsalflexion beschreibt eine Verschwenkung des Fußteils um die Schwenkachse des Gelenkes, bei der der Vorfuß-Bereich, also insbesondere die Zehen, nach oben, also in Richtung des Unterschenkels, bewegt werden. Die Plantarflexion ist die entgegengesetzte Bewegung.

Das Gelenk ist ein gedämpftes Gelenk. Es muss folglich eine Kraft oder ein Drehmoment ausgeübt werden, um die Dämpfung des Gelenkes zu überwinden und eine Verschwenkung des Fußteils relativ zum Unterschenkelteil zu erreichen. Derartige Änderungen sind in unterschiedlichster Form aus dem Stand der Technik bekannt. Bei einer hydraulischen Dämpfung beispielsweise wird beim Verschwenken des Fußteils relativ zum Unterschenkelteil eine Hydraulikflüssigkeit aus einem ersten Zylinder in einem zweiten Zylinder gedrückt. Dies geschieht durch eine Fluidverbindung, in der sich beispielsweise ein Drosselventil befindet. Dieses Ventil kann verstellt werden, wodurch ein schnellerer oder langsamerer Fluss durch die Fluidverbindung erreicht wird. Dadurch wird es vereinfacht oder erschwert, die beiden durch das Gelenk miteinander verbundenen Bauteile relativ zueinander zu verschwenken. Dadurch wird die Dämpfung eingestellt.

Die hier beschriebenen Gelenke verfügen vorzugsweise nicht über einen Antrieb, durch den beispielsweise das Fußteil relativ zu dem Unterschenkelteil verschwenkt werden kann. Diese Gelenke werden passive Gelenke genannt. Ein angetriebenes, also aktives Gelenk ist aus der US 10,314,723 B2 bekannt. Bei diesem Gelenk wird der Antrieb genutzt, um die Position der verschiedenen Bauteile der Prothese zu bewegen, um den gewünschten Verlauf des Krafteinleitungspunktes zu erreichen. Dies muss selbst bei sich nicht ändernden Gegebenheiten, wie beispielsweise dem Bewegungszustand des Trägers der Prothese oder dem Untergrund, über den der Träger geht, in jedem Schritt erneut geschehen, so dass das Verfahren sehr energieaufwändig ist und nur bei aktiven Prothesen anwendbar ist.

Die DE 603 09 685 T2, die den Oberbegriff des Anspruchs 1 offenbart, und die US 2002/0138153 A1 beschreiben gedämpfte Gelenke von orthopädietechnischen Einrichtungen, die magnetorheologisches System zum Dämpfen aufweisen. Die DE 20 2016 107 294 U1 beschreibt hingegen ein hydraulisches System.

Bei einem Prothesenfuß der hier beschriebenen Art kann das Unterschenkelteil sehr kurz ausgebildet sein. Es beinhaltet in diesem Fall insbesondere ein Anschlussstück, beispielsweise einen Pyramiden-Adapter, an dem ein Unterschenkelrohr oder eine sonstige Form eines künstlichen Unterschenkels angeordnet sein kann. Alternativ dazu kann das Unterschenkelteil auch länger ausgebildet sein und einstückig mit dem Unterschenkelrohr oder zumindest einem Teil eines Unterschenkelrohres ausgebildet sein. An dem dem Gelenk abgewandten Ende dieses Unterschenkel befindet sich dann ein weiteres Anschlussstück, beispielsweise einen Pyramiden-Adapter, an dem ein weiteres Prothesenelement, beispielsweise ein Unterschenkelrohr oder ein Prothesenknie-Gelenk angeordnet sein kann.

Es hat sich herausgestellt, dass es von Vorteil ist, die Dämpfung einzustellen, wenn beispielsweise der Träger des Prothesenfußes die Schuhe wechselt. Bei einem harten Schuh, der beispielsweise eine feste Ledersohle aufweist, ist eine weniger starke Dämpfung des Gelenks des Prothesenfußes notwendig, als bei einem sehr weichen Schuh, beispielsweise einem Laufschuh, einem Sportschuh oder einem Hausschuh. Aus dem Stand der Technik sind daher Prothesenfüße bekannt, die über eine Einstell-Einrichtung verfügen, durch die der Träger des Prothesenfu-βes selbstständig den Grad der Dämpfung des Prothesenfußes einstellen kann. Nachteilig ist jedoch, dass der Träger nur nach seinem Gefühl und Empfinden den Grad der Dämpfung einstellen kann und einer Reproduzierbarkeit der eingestellten Dämpfung für unterschiedliche Schuhe nicht gegeben ist. Insbesondere ist es nicht möglich, für unterschiedliche Schuhe die unterschiedlichen Dämpfungsgrade zu speichern.

Zudem sind Prothesenfüße bekannt, bei denen über Sensoren festgestellt wird, ob der Träger des Prothesenfußes bergauf oder bergab geht. In diesem Fall kann die Dämpfung automatisch angepasst werden, wobei auf dem Weg bergab die Dämpfung in Dorsalflexion-Richtung und beim Weg bergauf in Plantarflexion-Richtung erhöht wird. Nachteilig ist jedoch, dass dies für unterschiedliche Schuhe nicht möglich ist.

Eine aus dem Stand der Technik bekannte alternative Ausführungsform sieht vor, dass ein Dämpfungselement, das das Gelenk dämpft, ausgetauscht wird, wenn der Benutzer des Prothesenfußes den Schuh wechselt. Dies ist aufwendig und setzt voraus, dass der Benutzer die jeweils nötigen Dämpfungselemente bei sich führt.

Der Erfindung liegt daher die Aufgabe zugrunde, ein Verfahren zum Steuern des Prothesenfußes vorzuschlagen, bei dem auf unterschiedliche Schuhe mit gegebenenfalls unterschiedlichen Absatzhöhen sowie auf unterschiedliche Bewegungszustände reagiert werden kann, ohne dass der Träger des Prothesenfußes Bauteile auswechseln oder selbstständig Einstellungen vornehmen muss.

Die Erfindung löst die gestellte Aufgabe durch ein Verfahren zum Steuern eines Prothesenfußes der oben beschriebenen Art, dass die folgenden Schritte aufweist:
a) Erfassen von Messwerten, die Aussagen über ein Überrollverhalten des Prothesenfußes erlauben, mittels wenigstens eines Sensors,
b) Vergleichen der erfassten Messwerte und/oder wenigstens eines daraus bestimmten Parameters mit hinterlegten Sollwerten und
c) Anpassen der Dämpfung in Abhängigkeit des Vergleiches,
wobei die Messwerte mehrfach während eines Schrittzyklus erfasst werden, wobei vorzugsweise ein Verlauf der Messwerte über zumindest einen Teil des Schrittzyklus, bevorzugt den ganzen Schrittzyklus mit einem Verlauf der hinterlegten Sollwerte verglichen wird.

Das Überrollverhalten eines Prothesenfußes beschreibt, wie sich die Parameter des Prothesenfußes, durch die sich die Bewegung des Prothesenfußes beschreiben lässt, während des Überrollens, also während der Standphase eines Gangzyklus, in der der Prothesenfuß Kontakt mit dem Boden hat, verhalten. Diese Parameter können direkt messbare Messgrößen, beispielsweise ein Drehmoment, eine Kraft oder ein Winkel sein. Alternativ oder zusätzlich dazu können diese Parameter auch aus den Messgrößen bestimmbar sein.

Der Erfindung liegt die Erkenntnis zugrunde, dass ein gesunder Fuß sein eigenes Überrollverhalten sehr schnell so anpasst, dass das Überrollverhalten des Systems aus Fuß und Schuh nahezu konstant ist. Der Fuß gleicht die unterschiedlichen Überrollverhalten, die beispielsweise durch die unterschiedlich harten und unterschiedlich flexiblen Schuhe und Sohlen hervorgerufen werden, aus. Es ist somit nicht notwendig, eine Vielzahl unterschiedlicher Sollwerte desgleichen Messwertes oder Parameters zu hinterlegen, um für jeden Schuh, jede Absatz Höhe und jedes Bewegungsmuster passende Sollwerte bereitstellen zu können. Vielmehr sind die Sollwerte nahezu universell für alle Schuhe und Absatzhöhen sowie zumindest anteilig auch für unterschiedliche Bewegungsmuster verwendbar. Dabei ist selbstverständlich darauf zu achten, dass die jeweils ausgewählten Messwerte, die der wenigstens eine Sensor erfasst, und/oder der wenigstens eine daraus bestimmten Parameter, mit den hinterlegten Sollwerten vergleichbar sind. Die Sollwerte sind folglich Sollwerte für die jeweiligen Messwerte und/oder den wenigstens einen daraus bestimmten Parameter.

Erfindungsgemäß werden folglich derartige Messwerte, die Aussagen über dieses Überrollverhalten erlauben, mittels wenigstens eines Messwertes erfasst. Sie werden dann beispielsweise mit Sollwerten für diese erfassten Messwerte verglichen. Alternativ oder zusätzlich dazu werden aus den Messwerten ein oder mehrere Parameter bestimmt, die den mit Sollwerten für diesen wenigstens einen Parameter verglichen werden. In Abhängigkeit des Vergleiches wird die Dämpfung angepasst. Je nach Ergebnis des ausgeführten Vergleiches kann eine starke Anpassung, eine schwache Anpassung oder keine Anpassung vorgenommen werden.

Vorzugsweise wird die Dämpfung nur angepasst, wenn die Messwerte und/oder der wenigstens eine daraus bestimmten Parameter einen vorbestimmten Abstand von den Sollwerten überschreiten.

Bei dem Vergleich wird ein Abstand der Messwerte und/oder des wenigstens einen Parameters von diesem Sollwert ermittelt. Dies kann beispielsweise eine Differenz, ein Verhältnis, eine Standardabweichung oder eine sonstige Abweichung sein. Zuvor ist ein vorbestimmter Grenzwert, der sogenannte vorbestimmte Abstand, ermittelt und ebenfalls hinterlegt worden. Der ermittelte Abstand, der bei dem Vergleich zwischen Messwert und/oder Parameter mit dem Sollwert ermittelt wurde, wird nun mit dem vorbestimmten Abstand verglichen. Ist der ermittelte Abstand größer, kann die Dämpfung angepasst werden, wobei sich beispielsweise aus dem Vorzeichen des Abstandes bestimmt, ob die Dämpfung erhöht oder abgesenkt werden muss.

Erfindungsgemäß werden die Messwerte mehrfach während des Schrittzyklus erfasst. Da sie Aussagen über das Überrollverhalten, also das Verhalten von Parametern oder Messgrößen des Prothesenfußes über den Verlauf zumindest eines Teiles eines Schrittzyklus, bevorzugt über die Standphase, besonders bevorzugt über den gesamten Schrittzyklus, treffen sollen, ist es von Vorteil, den zeitlichen Verlauf der Messwerte über zumindest einen Teil des Schrittzyklusses, bevorzugt die Standphase, besonders bevorzugt den gesamten Schrittzyklus zu bestimmen. Sind die Messwerte selbst nicht mit Sollwerten zu vergleichen, muss aus den Messwerten und/oder dem zeitlichen Verlauf der Messwerte der jeweilige wenigstens eine Parameter und/oder dessen zeitlicher Verlauf ermittelt werden. Dabei kann es von Vorteil sein, zunächst den zeitlichen Verlauf der Messwerte zu ermitteln und aus diesem direkt den zeitlichen Verlauf des Parameters zu bestimmen. Alternativ dazu kann es von Vorteil sein, zu jedem Messzeitpunkt aus dem jeweiligen Messwert den wenigstens einen Parameter zu ermitteln und anschließend den zeitlichen Verlauf des Parameters zu bestimmen.

Bevorzugt wird die Plantardämpfung, also die einer Plantarflexion entgegenwirkende Dämpfung eingestellt. Dabei wird bevorzugt der Verlauf der Plantarflexion über den Knöchelwinkel und/oder den Unterschenkelwinkel angepasst. Der Knöchelwinkel ist der Winkel zwischen dem Unterschenkel und dem Fuß. Der Unterschenkelwinkel ist der Absolutwinkel des Unterschenkels, also beispielsweise der Winkel zwischen dem Unterschenkel und der Vertikalen. Die Vertikale ist die Richtung, in die das Schwerefeld der Erde wirkt. Bevorzugt wird der Verlauf zu Beginn des Fersenauftrittes, besonders bevorzugt vor Beginn des Fersenauftrittes angepasst. Eine weitere Einstellung im Verlauf des Schrittes wird bevorzugt nicht vorgenommen.

In einer Ausgestaltung des Verfahrens ist die angepasste Dämpfung beim Fersenauftritt, dem sogenannten heel-strike, vorhanden. Da dies der erste Teil der Standphase in einem Schrittzyklus ist, werden die Messwerte und/oder der wenigstens eine daraus bestimmte Parameter des vorangegangenen Schrittes verwendet. Vorzugsweise wird die Dämpfung während des weiteren Verlaufes der Standphase nicht erneut verändert oder angepasst oder wird auf der Grundlage der Messwerte und/oder des wenigstens einen daraus bestimmten Parameter des vorangegangenen Schrittes gesteuert und eingestellt. Dadurch wird der benötigte Rechenaufwand reduziert und das Verfahren energiesparend durchführbar. Es ist in einigen Ausgestaltungen des Verfahrens von Vorteil, wenn weitere Anpassungen während eines Schrittes erfolgen. Dies kann beispielsweise in einer Echtzeitsteuerung erfolgen.

Vorzugsweise beinhalten die Messwerte eine vertikale Kraft und ein Drehmoment am Gelenk, wobei aus den Messwerten vorzugsweise ein Krafteinleitungspunkt, besonders bevorzugt ein zeitlicher Verlauf des Krafteinleitungspunktes bestimmt wird. In der Standphase eines Schrittzyklus hat der Prothesenfuß Kontakt zum Boden. Dies beginnt mit dem Fersenauftritt. Von diesem Moment an nimmt zunächst die Belastung des Fußes und damit auch eine vertikale Kraft zu. Eine vertikale Kraft wirkt in der Richtung, in der auch die Gewichtskraft wirkt. Gleichzeitig wirkt auf das Gelenk des Prothesenfußes ein Drehmoment, und der Fuß führt eine Plantarflexion durch. Die Kontaktfläche zum Boden nimmt zu bis zu dem Moment, an dem der Fuß vollflächig aufliegt. Dabei kommt es zu einer Dorsalflexion, während der Unterschenkel relativ zum Fuß verschwenkt wird. Der Oberkörper bewegt sich weiter nach vorn. Auch wenn der Fuß in diesem Zeitraum vollflächig am Fußboden aufliegt wandert der Krafteinleitungspunkt weiter nach vorn. Die vertikale Kraft bleibt konstant, da der Fuß voll belastet ist und sich der andere Fuß in der Schwungphase befindet, während der er keinen Kontakt zum Boden hat. Es wirkt ein Drehmoment auf das Gelenk, dass eine Dorsalflexion hervorruft. Am Ende einer Standphase drückt der Fuß den Körper nach vorn, sodass die vertikale Kraft zunimmt und ein Drehmoment auf das Gelenk wirkt, dass erneut eine Plantarflexion bewirkt. Dieser Ablauf ist nahezu unabhängig von der Wahl des Schuhwerkes und der Bewegungsrichtung, beispielsweise auf einer Schräge bergauf oder bergab oder entlang einer Ebene. Die Stärke des Drehmoments und der vertikalen Kraft und insbesondere die Geschwindigkeit, mit der der Krafteinleitungspunkt nach vorn wandert hängt jedoch stark von diesen Parametern ab. Um für den Träger des Prothesenfußes und eine natürlich wirkende Bewegung zu gewährleisten wird die Dämpfung entsprechend angepasst.

Alternativ oder zusätzlich dazu wird der Krafteinleitungspunkt und/oder dessen zeitlicher Verlauf direkt gemessen und die Messwerte enthalten ihm. Dies kann beispielsweise erreicht werden, wenn der wenigstens eine Sensor eine Mehrzahl von Drucksensoren aufweist, die an einer Sohle des Fußteils angeordnet sind. Besonders bevorzugt handelt es sich um eine drucksensible Schicht, die an der Sohle des Fußteils angeordnet ist. Die Mehrzahl von Drucksensoren oder die drucksensible Schicht ist in der Lage, an unterschiedlichen Positionen an der Sohle des Fußteils den wirkenden Druck und damit die vertikale Kraft zu bestimmen. Da dies in einer Verteilung über die Sohle des Fußteils geschieht, braucht der Krafteinleitungspunkt nicht aufwendig aus den Messwerten bestimmt zu werden, sondern kann nahezu direkt aus den Messwerten abgelesen werden. Geschieht dies mehrfach während eines Gangzyklus kann der zeitliche Verlauf, also die Position des Krafteinleitungspunktes als Funktion der Zeit, bestimmt und gespeichert werden.

Unabhängig davon, wie der Krafteinleitungspunkt oder der zeitliche Verlauf des Krafteinleitungspunktes bestimmt wird, ist es von Vorteil, den zeitlichen Verlauf des Krafteinleitungspunktes durch einen Kreisabschnitt mit einem Mittelpunkt und einem Radius anzunähern. Bevorzugt werden dieser Mittelpunkt und Radius mit entsprechenden hinterlegten Sollwerten für Mittelpunkt und Radius verglichen. Das Annähern des zeitlichen Verlaufs des Krafteinleitungspunktes durch einen Kreisabschnitt kann durch nahezu alle bekannten fit-Verfahren, bei denen Messwerte mit einer Kurve gefittet werden, geschehen.

Herkömmlicherweise beträgt der Abstand zwischen dem Krafteinleitungspunkt und der Rotationsachse des Knöchelgelenkes ca. 0 bis 7 cm im Fersenbereich. Im Vorfußbereich liegt er zwischen 0 und 15 cm. Der Unterschenkelwinkel, also der absolut Winkel des Unterschenkels gegen die Vertikale, variiert zwischen -30 ° und +40 °, wobei 0° die Vertikale bezeichnet. Legt man dem optimalen Verlauf des Krafteinleitungspunkt einen Kreisabschnitt zugrunde, ergibt sich ein Radius von etwa 0,5 m.

Alternativ oder zusätzlich dazu beinhalten die Messwerte einen Unterschenkelwinkel und einen Fußwinkel, bevorzugt deren zeitlichen Verläufe. Besonders bevorzugt wird ein Verhältnis aus Unterschenkelwinkel und Fußwinkel und/oder dessen zeitlicher Verlauf bestimmt. Auch in dieser Ausgestaltung liegt der Erfindung die Erkenntnis zugrunde, das beispielsweise das Verhältnis aus Unterschenkelwinkel und Fußwinkel im zeitlichen Verlauf der Standphase des Gangzyklus nahezu unabhängig von der Wahl des Schuhwerkes und dessen Absatzhöhe ist. Der Fußwinkel und der Unterschenkelwinkel können dabei beispielsweise durch sogenannte Inertialsensoren bestimmt werden, die in der Lage sind, den Winkel gegen die Vertikale oder die Horizontale zu bestimmen. Die Vertikale ist dabei die Richtung, in der die Gravitation und die Gewichtskraft wirkt, während die Horizontale senkrecht auf der Vertikalen steht. Verändert sich das Verhältnis aus Unterschenkelwinkel und Fußwinkel beispielsweise zu schnell, kann die Dämpfung erhöht werden, um eine Änderung des Fußwinkel, die im Wesentlichen durch Verschwenken des Fußteils relativ zum Unterschenkelteil erzeugt wird, zu bremsen.

Vorzugsweise wird der Vergleich und gegebenenfalls die Anpassung der Dämpfung mehrfach, bevorzugt zeitlich äquidistant, während eines Teiles eines Schrittzyklus, bevorzugt über den ganzen Schrittzyklus, ausgeführt. Zu mehreren Zeitpunkten insbesondere während der Standphase wird folglich der Vergleich zwischen den Messdaten und/oder dem wenigstens einen aus ihnen bestimmten Parameter und den hinterlegten Sollwerten durchgeführt. Immer wenn bei diesem Vergleich der Abstand zwischen den Messwerten und/oder dem bestimmten Parameter und den hinterlegten Sollwerten größer als ein vorbestimmter Abstand ist, wird die Dämpfung angepasst. Auch dies kann, sofern notwendig, mehrfach während eines Schrittzyklus, bevorzugt mehrfach während der Standphase geschehen.

Vorzugsweise handelt es sich bei der Dämpfung um eine hydraulische und/oder eine magnetorheologische Dämpfung. Beide haben den Vorteil, dass sie sehr schnell einstellbar sind, da zur Anpassung der Dämpfung wenig oder bei der magnetorheologischen Dämpfung keine beweglichen Teile notwendig sind. Eine hydraulische Dämpfung kann die bereits beschriebene Ausführungsform sein, bei der beim Verschwenken des Fußteils relativ zum Unterschenkelteil eine Hydraulikflüssigkeit von einem Volumen in ein anderes Volumen verschoben wird. Dies geschieht durch eine Flüssigkeitsleitung oder Fluid-Verbindung, in der beispielsweise ein Drosselventil enthalten ist. Soll die Dämpfung erhöht werden, wird das Drosselventil weiter geschlossen, sodass der Strömungswiderstand in der Fluid-Verbindung erhöht wird. Sollte Dämpfung verringert werden, wird das Ventil weiter geöffnet, sodass der Strömungswiderstand verringert wird.

Bei einer magnetorheologischen Dämpfung wird eine Flüssigkeit oder ein Arbeitsmedium verwendet, dessen Fließfähigkeit, Viskosität und/oder Elastizität durch ein Magnetfeld beeinflussbar ist. Soll in diesem Fall beispielsweise die Dämpfung erhöht werden, wird ein Magnetfeld verstärkt, dem die magnetorheologische Flüssigkeit ausgesetzt ist. Dadurch wird die Viskosität verringert und somit eine Strömungswiderstand, der der Flüssigkeit entgegengesetzt wird, erhöht.

Vorzugsweise verfügt der Fußteil über wenigstens ein Federelement, dessen Federsteifigkeit angepasst wird, wenn die Messwerte einen vorbestimmten Abstand von den Sollwerten überschreiten. Dies ist eine zweite Möglichkeit, dass Überrollverhalten des Prothesenfußes zu modifizieren und an das gewünschte Verhalten anzupassen.

Die Erfindung löst die gestellte Aufgabe zudem durch einen Prothesenfuß mit einem Fußteil und einem Unterschenkelteil, die mittels eines Gelenkes miteinander verbunden sind, das eine Plantarflexion und eine Dorsalflexion erlaubt, wobei das Gelenk eine einstellbare Dämpfung auszeichnet. Der Prothesenfuß zeichnet sich dadurch aus, dass er über eine elektronische Datenverarbeitungseinrichtung verfügt, die eingerichtet ist zum Durchführen eines hier beschriebenen Verfahrens. Vorzugsweise verfügt der Prothesenfuß über einen elektronischen Datenspeicher, indem die Sollwerte hinterlegt sind. Über wenigstens einen Sensor, der Teil des Prothesenfußes sein kann, dies jedoch nicht muss, werden Messwerte erfasst, die an die elektronische Datenverarbeitungseinrichtung übermittelt werden. Diese vergleicht sie Messwerte entweder mit im elektronischen Datenspeicher hinterlegten Sollwerten oder ermittelt aus den Messwerten den zeitlichen Verlauf der Messwerte oder wenigstens einen Parameter oder dessen zeitlichen Verlauf.

Mithilfe der beiliegenden Figuren werden nachfolgend einige Ausführungsbeispiele der vorliegenden Erfindung näher erläutert. Es zeigen
- Figuren 1 bis 3 -: schematische Darstellungen von Verfahrensabläufe gemäß unterschiedlicher Ausführungsbeispiele der vorliegenden Erfindung und
- Figur 4 -: den Verlauf eines beispielhaften Messwertes.

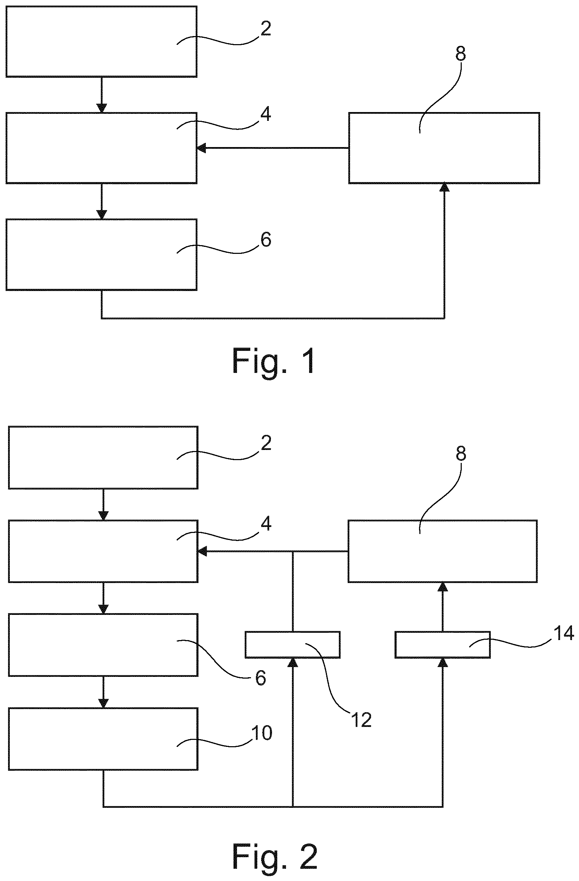

Figur 1 zeigt einen einfachen Verfahrensablauf. Zunächst werden in einem Festlegungsschritt 2 initiale Dämpfungswerte für die Dämpfung des Gelenkes des Prothesenfußes festgelegt. Mit diesen initialen Dämpfungswerten wird zumindest der erste Schritt, der mit dem Prothesenfuß zurückgelegt wird, durchgeführt.

In einem Erfassungsschritt 4 werden die Messwerte mittels des wenigstens einen Sensors, der an dem Prothesenfuß oder einem daran befestigten Element angeordnet ist, erfasst. Diese Messwerte betreffen beispielsweise den Verlauf eines Krafteinleitungspunktes als Funktion des Unterschenkelwinkels und/oder des Knöchelwinkels. Um den Verlauf bestimmen zu können, muss die Position des Krafteinleitungspunktes mehrfach nacheinander zumindest über einen Abschnitt des Schrittes erfasst werden. Vorzugsweise wird mit der Messung beim Fersenauftritt begonnen und die Messungen erstrecken sich vorzugsweise über die gesamte Plantarflexionsphase des Schrittes.

In einem Vergleichsschritt 6 wird der so gemessene Verlauf des Krafteinleitungspunktes mit einem Soll-Verlauf verglichen. Es wird dabei ein Abstand zwischen dem gemessenen Verlauf und dem Soll-Verlauf festgestellt und die Abweichung quantifiziert.

Auf der Basis dieses Abstandes wird in einem Anpassungsschritt 8 die Dämpfung angepasst, vorzugsweise bevor der nächste Schritt begonnen wird. Danach wird im nächsten Schritt der nächste Erfassungsschritt 4 durchgeführt und erneut die jeweiligen Messwerte, vorliegend also der Verlauf des Krafteinleitungspunktes erfasst.

Figur 2 zeigt ein ähnliches Verfahren. Auch hier werden im Festlegungsschritt 2 initiale Dämpfungswerte für das Gelenk des Prothesenfußes festgelegt. Anschließend werden im Erfassungsschritt 4 die Messwerte erfasst. Diese werden im Vergleichsschritt 6 mit entsprechenden Soll-Daten verglichen. Anders als im in Figur 1 gezeigten Ausführungsbeispiel wird in einem zusätzlichen Prüfschritt 10 geprüft, ob die im Vergleichsschritt 6 ermittelte Abweichung, also der Abstand der Messwerte/oder des wenigstens einen daraus bestimmten Parameters von den hinterlegten Soll-Werten, einen vorbestimmten Grenzwert überschreitet. Ist dies nicht der Fall, wird entlang des "Nein"-Pfades 12 keine Anpassung der Dämpfung vorgenommen. Die Abweichung ist zu klein. Stattdessen wird im nächsten Schritt, den der Träger mit dem Prothesenfuß durchführt, erneut ein Erfassungsschritt 4 durchgeführt.

Ist hingegen der bestimmte Abstand größer als der vorbestimmte Grenzwert wird entlang des "Ja"-Pfades 14 zum Anpassungsschritt 8 übergeleitet, sodass die Dämpfung des Gelenkes angepasst wird.

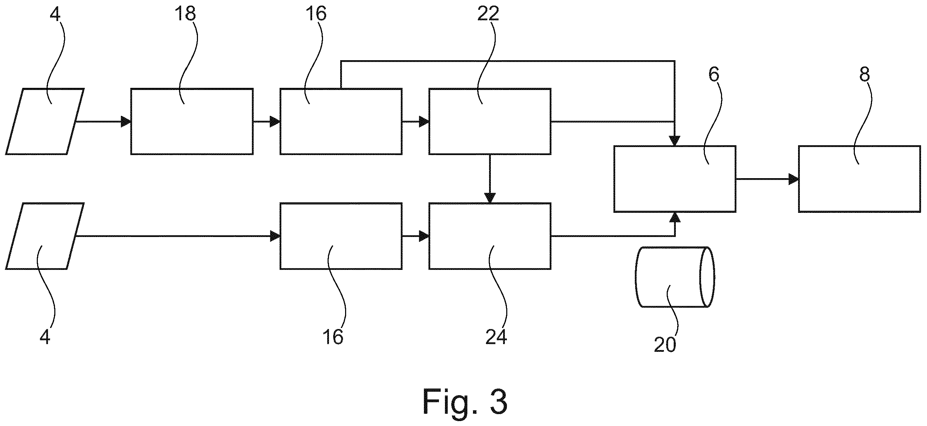

Figur 3 zeigt eine detailliertere Darstellung des Verfahrens. Der Festlegungsschritt 2 ist aus Übersichtlichkeitsgründen weggelassen worden. Im Erfassungsschritt 4 werden Messwerte erfasst, die beispielsweise Sensordaten sind. In Figur 3 sind zwei Erfassungsschritte 4 dargestellt, die nicht zwangsläufig beide durchgeführt werden müssen. Sie beschreiben unterschiedliche Verfahren, die alternativ oder zusätzlich zueinander durchgeführt werden können. Die aus dem unteren Erfassungsschritt 4 erfassten Messwerte werden in einem Aufzeichnungsschritt 16 über zumindest einen Teil der Standphase des Schrittes, vorzugsweise jedoch über die gesamte Standphase des Schrittes aufgezeichnet.

Die aus dem oberen Erfassungsschritt 4 entstehenden Messwerte werden in einem Umrechnungsschritt 18 in wenigstens einen Parameter umgerechnet, der auf den Messwerten basiert. Im nächsten Verfahrensschritt wird dann der so ermittelte Parameter über zumindest einen Teil der Standphase des Schrittes, vorzugsweise jedoch über die gesamte Standphase des Schrittes aufgezeichnet. Auch hierbei handelt es sich folglich um einen Aufzeichnungsschritt 16.

Nach diesem Aufzeichnungsschritt 16 kann der ermittelte und aufgezeichnete Parameter im Vergleichsschritt 6 direkt mit Soll-Werten verglichen werden, die als Referenz-Werte aus einem elektronischen Datenspeicher 20, der nur schematisch dargestellt ist, bereitgestellt werden. Anschließend erfolgt im Anpassungsschritt 8 die auf der Basis dieses Vergleiches vorzunehmende Anpassung der Dämpfung. Alternativ dazu kann in einem zweiten Umrechnungsschritt 22 ein weiterer Parameter aus dem Verlauf des Kennwertes oder des bisher errechneten Parameters erstellt werden. Ist dies der Fall wird anschließend dieser Verlauf des Kennwertes oder Parameters im Vergleichsschritt 6 verglichen und auf der Basis dieses Vergleiches im Anpassungsschritt 8 die Anpassung der Dämpfung vorgenommen.

In einer bevorzugten Ausgestaltung des Verfahrens werden die aus dem unteren Erfassungsschritt 4 erfassten Messwerte, die im unteren Aufzeichnungsschritt 16 aufgezeichnet wurden, gemeinsam mit den aus dem zweiten Umrechnungsschritt 22 bestimmten Parametern aufbereitet, indem beispielsweise ein Phasendiagramm 24 erstellt wird. Auch dieses kann dann im Vergleichsschritt 6 mit Sollwerten aus dem elektronischen Datenspeicher 20 verglichen werden.

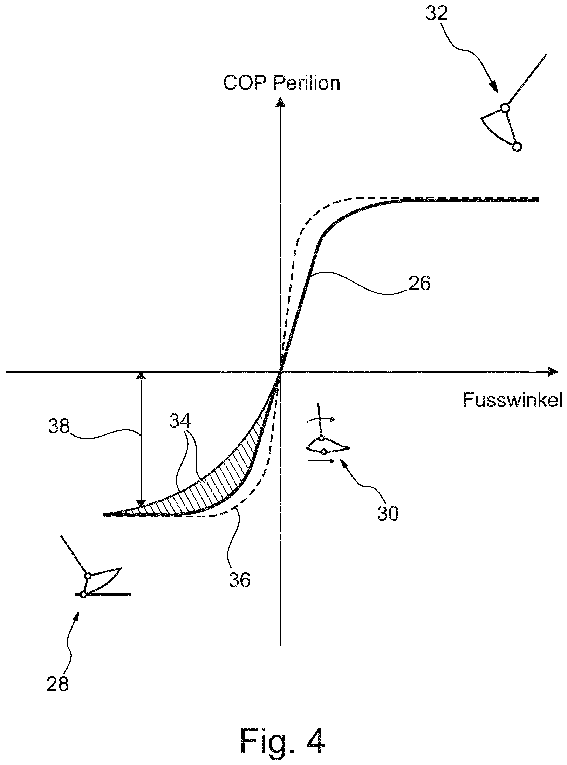

Figur 4 zeigt schematisch einen Verlauf eines Messwertes. Aufgetragen ist die Position des Krafteinleitungspunktes auf der vertikalen Y-Achse und der Fußwinkel, also der Winkel zwischen dem Fußteil und dem Boden, auf dem der Träger der Prothese geht, auf der horizontalen X-Achse. Eine Soll-Kurve 26 zeigt den gewünschten Verlauf. Während eines Schrittes beginnt der Verlauf im linken unteren Quadrant. Der Krafteinleitungspunkt (COP) ist im Bereich der Ferse und beginnt beim Fersenauftritt. Dies wird durch das erste Piktogramm 28 dargestellt. Verfolgt man die Soll-Kurve zu steigendem Fußwinkel erkennt man, dass der Krafteinleitungspunkt zunächst an der Ferse bleibt, bevor er im gezeigten Diagramm nach oben, also in Richtung auf den Vorfuß, wandert.

Im Ursprung des Diagrammes liegt der Punkt, an dem der Fuß vollständig auf dem Boden aufliegt und der Unterschenkel über den Fuß hinwegschwingt. Dies ist durch das zweite Piktogramm 30 schematisch dargestellt. Der Krafteinleitungspunkt wandert mit steigendem Fußwinkel weiterhin Richtung Vorfuß bevor er im Bereich der Zehen bleibt, bis diese sich vom Boden lösen. Diese Situation ist im dritten Piktogramm 32 dargestellt

Unterschiedliche gemessene Kurven sind durch die dünn dargestellte durchgezogene Linie 34 und die gestrichelte dargestellte Linie 36 dargestellt. Bei der Linie 34 bewegt sich der Krafteinleitungspunkt früher als bei der Soll-Kurve von der Ferse des Fußes weg, der Fuß plantarflektiert nicht ausreichend. Ein Fersenhebel, der durch den Doppelpfeil 38 dargestellt ist, wird dabei reduziert. Um diese Abweichung von der Soll-Kurve zu beheben, wird die Dämpfung verringert, also der einer Bewegung entgegenstehende Widerstand verkleinert. Damit kann die Linie 34 in Richtung auf die Soll-Kurve bewegt werden. Der Fuß plantarflektiert nun schneller.

Die gestrichelte Linie 36 weicht in die andere Richtung von der Soll-Kurve ab. Hier ist die Dämpfung zu weich, sodass der Fuß zu schnell plantarflektiert und der Krafteinleitungspunkt daher mit zunehmendem Fußwinkel zunächst nicht wandert und erst bei größerem Fußwinkel als gewünscht von der Ferse in Richtung Vorfuß wandert. In diesem Fall sollte die Dämpfung erhöht werden.

### Bezugszeichenliste:

- 2: Festlegungsschritt
- 4: Erfassungsschritt
- 6: Vergleichsschritt
- 8: Anpassungsschritt
- 10: Prüfschritt
- 12: "Nein"-Pfad
- 14: "Ja"-Pfad
- 16: Aufzeichnungsschritt
- 18: Umrechnungsschritt
- 20: elektronischer Datenspeicher
- 22: zweiter Umrechnungsschritt
- 24: Phasendiagramm
- 26: Soll-Kurve
- 28: erstes Piktogramm
- 30: zweites Piktogramm
- 32: drittes Piktogramm
- 34: durchgezogene Linie
- 36: gestrichelte Linie
- 38: Fersenhebel

## Patentansprüche

1. Verfahren zum Steuern eines Prothesenfußes, der ein Fußteil und ein Unterschenkelteil aufweist, die mittels eines Gelenkes miteinander verbunden sind, das eine Plantarflexion und eine Dorsalflexion erlaubt, wobei das Gelenk eine einstellbare Dämpfung aufweist,
wobei das Verfahren folgende Schritte aufweist:
a) Erfassen von Messwerten, die Aussagen über ein Überrollverhalten des Prothesenfußes erlauben, mittels wenigstens eines Sensors,
**gekennzeichnet durch**
b) Vergleichen der erfassten Messwerte und/oder wenigstens eines daraus bestimmten Parameters mit hinterlegten Sollwerten und
c) Anpassen der Dämpfung in Abhängigkeit des Vergleiches,
wobei die Messwerte mehrfach während eines Schrittzyklus erfasst werden,
wobei ein Verlauf der Messwerte über zumindest einen Teil des Schrittzyklus mit einem Verlauf der hinterlegten Sollwerte verglichen wird.

2. Verfahren nach Anspruch 1, **dadurch gekennzeichnet, dass** die Dämpfung nur angepasst wird, wenn die Messwerte und/oder der wenigstens eine daraus bestimmte Parameter einen vorbestimmten Abstand von den Sollwerten überschreiten.

3. Verfahren nach Anspruch 1 oder 2, **dadurch gekennzeichnet, dass** ein Verlauf der Messwerte über den ganzen Schrittzyklus mit einem Verlauf der hinterlegten Sollwerte verglichen wird

4. Verfahren nach einem der vorstehenden Ansprüche, **dadurch gekennzeichnet, dass** ein Verlauf der Plantardämpfung über den Knöchelwinkel und/oder den Unterschenkelwinkel, angepasst wird, wobei der Verlauf vorzugsweise zu Beginn des Fersenauftrittes, besonders bevorzugt vor Beginn des Fersenauftrittes, angepasst wird und im weiteren Verlauf eines Schrittes keine weitere Anpassung des Verlaufes erfolgt.

5. Verfahren nach einem der vorstehenden Ansprüche, **dadurch gekennzeichnet, dass** die Messwerte eine vertikale Kraft und ein Drehmoment am Gelenk beinhalten wobei aus den Messwerten vorzugsweise ein Krafteinleitungspunkt, besonders bevorzugt ein zeitlicher Verlauf des Krafteinleitungspunktes bestimmt wird.

6. Verfahren nach einem der vorstehenden Ansprüche, **dadurch gekennzeichnet, dass** die Messwerte den Krafteinleitungspunkt und/oder dessen zeitlichen Verlauf beinhalten, und der wenigstens eine Sensor eine Mehrzahl von Drucksensoren, bevorzugt eine drucksensible Schicht an einer Unterseite einer Sohle des Fußteils aufweist.

7. Verfahren nach Anspruch 5 oder 6, **dadurch gekennzeichnet, dass** der zeitliche Verlauf des Krafteinleitungspunktes durch einen Kreisabschnitt mit einem Mittelpunkt und Radius angenähert wird, die mit einem hinterlegten Mittelpunkt und/oder Radius verglichen werden.

8. Verfahren nach einem der vorstehenden Ansprüche, **dadurch gekennzeichnet, dass** die Messwerte ein Unterschenkelwinkel und ein Fußwinkel, bevorzugt deren zeitlichen Verläufe sind, wobei besonders bevorzugt ein Verhältnis aus Unterschenkelwinkel und Fußwinkel und/oder dessen zeitlicher Verlauf bestimmt wird.

9. Verfahren nach einem der vorstehenden Ansprüche, **dadurch gekennzeichnet, dass** der Vergleich und gegebenenfalls die Anpassung der Dämpfung mehrfach, bevorzugt zeitlich äquidistant, während eines Teiles eines Schrittzyklus, bevorzugt über den ganzen Schrittzyklus, ausgeführt wird.

10. Verfahren nach einem der vorstehenden Ansprüche, **dadurch gekennzeichnet, dass** des sich bei der Dämpfung um eine hydraulische und/oder eine magnetorheologische Dämpfung handelt.

11. Verfahren nach einem der vorstehenden Ansprüche, **dadurch gekennzeichnet, dass** der Fußteil wenigstens ein Federelement aufweist, dessen Federsteifigkeit angepasst wird, wenn die Messwerte einen vorbestimmten Abstand von den Sollwerten überschreiten.

12. Prothesenfuß mit einem Fußteil und einem Unterschenkelteil, die mittels eines Gelenkes miteinander verbunden sind, das eine Plantarflexion und eine Dorsalflexion erlaubt, wobei das Gelenk eine einstellbare Dämpfung aufweist, **gekennzeichnet durch** eine elektronische Datenverarbeitungseinrichtung, die eingerichtet ist zum Durchführen eines Verfahrens nach einem der vorstehenden Ansprüche.

## Claims

1. A method for controlling a prosthetic foot that has a foot part and a lower leg part which are connected to each other by means of a joint that allows a plantar flexion and a dorsal flexion, the damping behavior of the joint being adjustable,
wherein the method comprises the following steps:
a) detecting measured values which allow for statements to be made about the rollover behavior of the prosthetic foot by means of at least one sensor,
**characterized in that**
b) comparing the detected measured values and/or at least one parameter determined from said values with stored target values, and
c) adjusting the damping behavior depending on the comparison,
wherein the measured values are detected multiple times during a step cycle,
wherein a course of the measured values across at least one part of the step cycle is compared with a course of the stored target values.

2. The method according to claim 1, **characterized in that** the damping behavior is only adjusted when the difference between measured values and/or the at least one parameter determined from said values and the target values exceed a predetermined gap.

3. The method according to claim 1 or 2, **characterized in that** a course of the measured values across the entire step cycle, is compared with a course of the stored target values.

4. The method according to one of the preceding claims, **characterized in that** a course of the plantar damping as a function of the ankle angle and/or the lower leg angle is adjusted, wherein the course is preferably adjusted at the start of the heel strike, particularly preferably before the start of the heel strike, and no further adjustment of the course occurs over the remaining course of the step.

5. The method according to one of the preceding claims, **characterized in that** the measured values include a vertical force and a torque on the joint, wherein preferably a force application point, particularly preferably a course of the force application point, is determined from the measured values.

6. The method according to one of the preceding claims, **characterized in that** the measured values contain the force application point and/or its chronological profile, and the at least one sensor comprises a plurality of pressure sensors, preferably a pressure-sensitive layer on a lower side of a sole of the foot part.

7. The method according to claim 5 or 6, **characterized in that** the chronological profile of the force application point is approximated by a segment of a circle with a center point and radius, which are compared with a stored center point and/or radius.

8. The method according to one of the preceding claims, **characterized in that** the measured values are a lower leg angle and a foot angle, preferably their chronological profiles, wherein it is especially preferable if a ratio of lower leg angle to foot angle and/or its chronological profile is determined.

9. The method according to one of the preceding claims, **characterized in that** the comparison and, if necessary, the adjustment of the damping behavior is performed multiple times, preferably at equidistant intervals, during part of a step cycle, preferably across the entire step cycle.

10. The method according to one of the preceding claims, **characterized in that** the damping is a hydraulic and/or magnetorheological damping.

11. The method according to one of the preceding claims, **characterized in that** the foot part has at least one spring element, the spring stiffness of which is adjusted when the measured values exceed a predetermined distance from the target values.

12. A prosthetic foot with a foot part and a lower leg part that are connected to each other via a joint which allows a plantar flexion and a dorsal flexion, the damping behavior of the joint being adjustable, **characterized by** an electronic data processing device that is configured to conduct a method according to one of the preceding claims.

## Revendications

1. Procédé de commande d'un pied prothétique comprenant une partie de pied et une partie de jambe reliées l'une à l'autre au moyen d'une articulation qui permet une flexion plantaire et une flexion dorsale, l'articulation présentant un amortissement réglable,
le procédé présentant les étapes suivantes consistant à :
a) saisir des valeurs de mesure qui permettent des informations sur un comportement de retournement du pied prothétique, au moyen d'au moins un capteur,
**caractérisé par**
b) la comparaison des valeurs de mesure saisies et/ou d'au moins un paramètre, déterminé à partir de celles-ci, à des valeurs de consigne enregistrées, et
c) l'adaptation de l'amortissement en fonction de la comparaison,
sachant que les valeurs de mesure sont saisies plusieurs fois pendant un cycle de marche, et une évolution des valeurs de mesure sur au moins une partie du cycle de marche est comparée à une évolution des valeurs de consigne enregistrées.

2. Procédé selon la revendication 1,
**caractérisé en ce que** l'amortissement n'est adapté que si les valeurs de mesure et/ou ledit au moins un paramètre, déterminé à partir de celles-ci, dépasse(nt) un écart prédéterminé par rapport aux valeurs de consigne.

3. Procédé selon la revendication 1 ou 2,
**caractérisé en ce qu'**une évolution des valeurs de mesure sur tout le cycle de marche est comparée à une évolution des valeurs de consigne enregistrées.

4. Procédé selon l'une des revendications précédentes,
**caractérisé en ce qu'**une évolution de l'amortissement plantaire est adaptée en fonction de l'angle de la cheville et/ou de l'angle de la jambe, sachant que l'évolution est adaptée de préférence au début de l'appui sur le talon, de manière particulièrement préférée avant le début de l'appui sur le talon, et aucune autre adaptation de l'évolution n'est effectuée au cours d'une poursuite de la marche.

5. Procédé selon l'une des revendications précédentes,
**caractérisé en ce que** les valeurs de mesure comprennent une force verticale et un couple au niveau de l'articulation, sachant qu'un point d'application de la force, de préférence une évolution dans le temps du point d'application de la force, est déterminé(e) à partir des valeurs de mesure.

6. Procédé selon l'une des revendications précédentes,
**caractérisé en ce que** les valeurs de mesure comprennent le point d'application de la force et/ou son évolution dans le temps, et ledit au moins un capteur comprend une pluralité de capteurs de pression, de préférence une couche sensible à la pression, sur une face inférieure de la semelle de la partie de pied.

7. Procédé selon la revendication 5 ou 6,
**caractérisé en ce que** l'évolution dans le temps du point d'application de la force est approchée par un segment de cercle ayant un centre et un rayon qui sont comparés à un centre et/ou un rayon enregistré.

8. Procédé selon l'une des revendications précédentes,
**caractérisé en ce que** les valeurs de mesure sont un angle de la jambe et un angle du pied, de préférence leurs évolutions dans le temps, sachant que de manière particulièrement préférée un rapport est déterminé entre l'angle de la jambe et l'angle du pied et/ou leurs évolutions dans le temps.

9. Procédé selon l'une des revendications précédentes,
**caractérisé en ce que** la comparaison et, le cas échéant, l'adaptation de l'amortissement sont effectuées plusieurs fois, de préférence de manière équidistante dans le temps, pendant une partie d'un cycle de marche, de préférence pendant tout le cycle de marche.

10. Procédé selon l'une des revendications précédentes,
**caractérisé en ce que** l'amortissement est un amortissement hydraulique et/ou magnétorhéologique.

11. Procédé selon l'une des revendications précédentes,
**caractérisé en ce que** la partie de pied comprend au moins un élément élastique dont la raideur est adaptée si les valeurs de mesure dépassent un écart prédéterminé par rapport aux valeurs de consigne.

12. Pied prothétique comprenant une partie de pied et une partie de jambe reliées l'une à l'autre au moyen d'une articulation qui permet une flexion plantaire et une flexion dorsale, l'articulation présentant un amortissement réglable,
**caractérisé par** un dispositif électronique de traitement de données conçu pour mettre en œuvre un procédé selon l'une des revendications précédentes.
